**Europäisches Patentamt**

**European Patent Office**

**Office européen des brevets**

(19)

(11) Numéro de publication: **0 080 705**
**B1**

(12) **FASCICULE DE BREVET EUROPEEN**

(45) Date de publication du fascicule du brevet: 30.04.86

(51) Int. Cl.⁴: **G 01 K 13/00, A 61 B 10/00**

(21) Numéro de dépôt: 82110903.0

(22) Date de dépôt: 25.11.82

(54) Instrument électronique pour le contrôle et le traitement de l'infertilité féminine.

(30) Priorité: 01.12.81 CH 7692/81

(43) Date de publication de la demande:
08.06.83 Bulletin 83/23

(45) Mention de la délivrance du brevet:
30.04.86 Bulletin 86/18

(84) Etats contractants désignés:
AT BE DE FR GB IT SE

(56) Documents cités:
EP-A-0 022 061
EP-A-0 031 251
GB-A-2 045 480
US-A-4 031 365
US-A-4 151 831

(73) Titulaire: ARES N.V. Amsterdam NL
Swiss Branch 2, Chemin des Mines
CH-1202 Genève (CH)

(72) Inventeur: Nessi, Pierre
2, Chemin des Mines
CH-1202 Genève (CH)
Inventeur: Crausaz, Jacques
4, rue du Musée
CH-1700 Fribourg (CH)

(74) Mandataire: Maspoli, Renato A., Dipl.-
Chem.Ing.ETH
Patentanwälte R.A. Maspoli und Partner
Promenadengasse 18
CH-8001 Zürich (CH)

Courier Press, Leamington Spa, England.

EP 0 080 705 B1

## Description

La présente invention se réfère à un instrument électronique pour le contrôle et le traitement de l'infertilité féminine au moyens de la mesure de la température corporelle basale (TCB).

Le cycle menstruel de référence (normal) d'une femme fertile est caractérisé par une courbe de température (TCB) biphasique, c'est-à-dire présentant une élévation de température, quelques heures après l'ovulation, élévation qui se situe généralement vers le milieu du cycle.

La TCB est obtenue en mesurant, par exemple à l'aide d'un thermomètre conventionnel, la température corporelle chaque matin avant le lever; la valeur de cette température est ensuite reportée manuellement sur une feuille graphique.

Dans les cas d'infertilité la courbe TCB est généralement modifiée; c'est la raison pour laquelle le médecin gynécologue commence usuellement ses investigations, chez une patiente souffrante d'infertilité, par l'analyse de 2 ou 3 cycles TCB.

Dans un certain nombre de cas d'infertilité, la courbe TCB n'est plus biphasique, mais monophasique ou irrégulière; ceci laisse supposer que l'ovulation n'a plus lieu ou qu'elle ne se produit que de manière intermittente. En face d'une telle situation, comme dans un certain nombre d'autres, le traitement utilisé par le gynécologue consiste en une thérapie hormonale substitutive, qui a pour but de reinitier un cycle normal chez la patiente, cycle caractérisé à nouveau par un système de TCB biphasique. Pour démontrer une certaine efficacité, le traitement hormonal substitutif doit avoir lieu à certains jours fixés avec précision le long du cycle de la patiente; les intervalles entre les jours de traitement ainsi que l'intervalle entre le traitement et l'observation des résultats de la thérapie doivent, eux aussi, avoir lieu à des dates fixées avec précision.

La présente invention consiste en un instrument électronique capable de contrôler lesdites données fondamentales pour un traitement efficace de l'infertilité féminine.

L'invention selon EP—A—0 031 251 consiste, selon description et revendications, en un instrument électronique capable de mesurer la température corporelle, d'en calculer la valeur moyenne d'un nombre de jour prédéterminé et de comparer ladite moyenne avec la valeur d'une nouvelle mesure. Le but du procédé est de confirmer l'élévation de la température corporelle dûe à l'ovulation.

L'appareil et la méthode selon ledit EP—A—0031251 ne se prête pas à l'analyse du cycle menstruel pour un traitement d'infertilité.

L'indicateur de fertilité selon le US—A—4 151 831 apporte des moyens pour lire la température corporelle stabilisée automatiquement. Cela implique une longue durée de l'application des moyens de mesure (colonne 2, lignes 43 à 45). L'appareil comprend surtout des moyens pour contrôler si la température mesurée s'est stabilisée et pour déterminer et indiquer les états fertilité/infertilité.

Il n'y a, dans l'appareil selon le brevet US—A—4151831 sus-indiqué, ni des moyens pour remplacer une température qu'on a oublié de mesurer ni des moyens pour comparer les deux moyennes de températures correspondentes aux phases folliculaire et lutéale. Cela veut dire que l'appareil selon US—A—4 151 831 ne se prête pas au contrôle et au traitement de l'infertilité féminine.

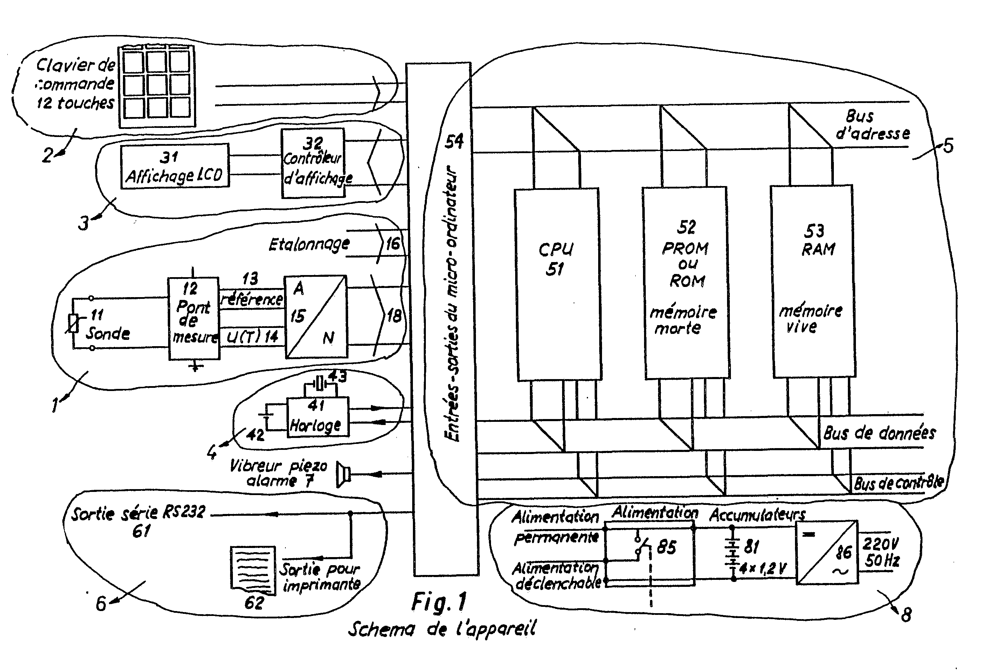

Selon l'invention l'instrument électronique pour le contrôle et le traitement de l'infertilité féminine, illustré à titre d'exemple dans la figure 1 du dessin annexé, comprend une sonde thermistor pour la mesure de la température, un circuit d'entrée, reliant la sonde à un microprocesseur 5, comportant un convertisseur analogique-numérique 15 permettant de transformer la variation de résistance du thermistor en bits, un circuit d'horloge permettant de n'accepter qu'une température par jour dite température corporelle basale (TCB), un dispositif acoustique signalant le début et la fin de la prise de température, le microprocesseur étant agencé pour faire une analyse continue et globale de la courbe mensuelle des températures corporelles basales, et un display. Ledit instrument électronique est caractérisé en ce que le microprocesseur comporte une mémoire RAM pour le stockage non volatile de 100 températures corporelles basales, en ce que le dispositif acoustique est un vibreur piezo-électrique, en ce que le display est un display LCD commandé par l'extérieur et en ce que ledit instrument électronique comporte des moyens discriminateurs pour remplacer une température corporelle basale manquante correspondant à un jour sauté ou une température corporelle basale trop élevée correspondant à un jour de fièvre par la moyenne des températures corporelles basales des 2 jours entourant le jour sauté ou le jour de fièvre et des moyens comparateurs permettant de comparer, dès le 8ème jour du cycle, deux moyennes de température: une première fois la moyenne $M_F$ de la phase folliculaire supposée, dans les jours 4 et 5 du cycle, et la moyenne $M_L$ de la phase lutéale supposée, dans les jours 6, 7 et 8 du cycle, les 3 premiers jours du cycle n'étant pas pris en considération, ces moyens comparateurs étant tels que ce mécanisme de comparaison se poursuive d'autres fois à chaque prise de température jusqu'à ce que $D = M_L - M_F$ dépasse un niveau prédéterminé, $M_L$ correspondant à la moyenne des trois dernières températures corporelles basales et $M_F$ à la moyenne des autres températures à partir du quatrième jour, après quoi on considère d'être en présence de la seconde phase du cycle, l'antipénultième jour du cycle étant alors considéré comme l'$ETO_s$, s'est-à-dire l'"Estimated Time of Ovulation".

L'instrument selon l'invention peut comporter, en outre, des moyens permettant, à la fin du cycle, de classifier la courbe mensuelle dans l'une des 3 catégories suivants:

(i) courbe mensuelle monophasique ou irrégulière,

(ii) courbe mensuelle biphasique supposée,

(iii) courbe mensuelle biphasique certaine des températures corporelles basales

et des moyens pour classifier les courbes supposées biphasiques en "courbes monophasique" ou en "courbes biphasiques".

L'instrument selon l'invention peut comporter, en outre, des moyens permettant de calculer l'ETO$_f$, c'est-à-dire l'"Estimated Time of Ovulation—final" dans le cas d'une courbe classée "biphasique" et des moyens pour comparer l'ETO$_s$ et l'ETO$_f$ afin d'accepter ou de rejeter l'ETO, c'est-à-dire l'"Estimated Time of Ovulation".

L'instrument selon l'invention peut comporter, en outre, des moyens d'afficher sur le display (LCD 31 le jour d'ETO, la longueur du cycle, ainsi que des longueurs des phases post-et prémenstruelles, et des moyens permettant de mémoriser ces valeurs, des moyens pour faire défiler sur le display LCD chacune des 100 températures journalières mémorisées, accompagnées des pointeurs de début du cycle, c'est à dire ler jour des règles, des pointeurs de jour sauté et des pointeurs de jour de fièvre et des sorties normalisées permettant

le transfert automatique des températures journalières mémorisées dans un ordinateur plus puissant à des fins d'analyse détaillée,

le transfer et l'impression automatique des températures journalières mémorisées sur une imprimante,

le tranfert et l'expression graphique de la courbe mensuelle sur une imprimante,

l'impression du code de la patiente, du jour d'ETO, de longueur du cycle et des phases post-et prémenstruelles à l'aide de l'imprimante et

le transfer et l'impression du planning journalier personalisé pour la thérapie hormonale.

L'instrument selon l'invention comprend normalement 12 boutons pressoirs permettant, grâce à un nombre de code défini, d'accéder

avec le code AAA à la fonction TCB, c'est-à-dire mesuré de la température corporelle basale,

avec le code BBB à la fonction T, c'est-à-dire traitement

avec le code CCC à la fonction thermomètre, d'est-à-dire simple mesure de la température,

avec le code DDD à la fonction horloge, c'est-à-dire affichage des heures, minutes et secondes et

avec le code EEE à la fonction calendrier, c'est-à-dire affichage des années, mois et jours.

L'instrument selon l'invention peut comprendre, en outre, des moyens permettant à l'instrument de fonctionner comme un thermomètre à maximum, un signal acoustique destiné à rappeler à l'utilisatrice que la mesure de température doit avoir lieu, et à lui signaler la fin de ladite mesure et un display LCD indiquant, en plus, à l'utilisatrice le numéro du jour du cycle et sa température associée.

Le circuit d'horloge de l'instrument selon l'invention permet de n'accepter une température corporelle basale qu'à l'intérieur d'un intervalle de temps fixé, dit fenêtre matinale, et de n'accepter cette température corporelle basale que si elle est située entre 35,0 et 37,5°C.

L'instrument analyse la courbe TCB de manière continue: à l'aide d'un algorithme mathématique no. 1, il est à même de détecter une élévation de température présupposant l'incidence d'une ovulation. A la fin du cycle—indiqué manuellement lors de l'arrivée des prochains règles—le calculateur analyse la courbe TCB mensuelle dans sa totalité et définit, à l'aide d'un algorithme mathématique no. 2, un jour d'ovulation qui corrobore ou non le jour d'ovulation défini lors de l'entrée des températures journalières. Puis, dans les cas où l'ovulation est connue et confirmée, l'instrument calcule et affiche toutes les températures individuelles, la longueur du cycle, le jour estimé pour l'ovulation, la longueur des phases post- et prémenstruelles; toutes ces données sont stockées dans le calculateur et seront utilisées pour des prévisions sur les cycles suivants. Enfin, utilisé en mode T (traitement hormonal destiné à réinitier un cycle menstruel normal) l'appareil affiche, à partir du premier jour de traitement (défini par le médicin gynécologue) les jours où les prises hormonales doivent avoir lieu, ainsi que les jours prévus pour une visite chez le médecin à des fins d'observation et/ou d'analyses sanguines.

Il est donc fourni: un appareil électronique pour la mesure de la TCB, comprenant une sonde NTC (thermistor) pour l'acquisition de la température, un circuit d'entré et de transformation de la température en bits, un microprocesseur pour le stockage et le traitement de l'information, un display LCD pour l'affichage des résultats. L'instrument comporte également une horloge, permettant de fournir à tout instant le jour et l'heure et n'autorisant qu'une mesure de TCB par jour à l'intérieur d'une fenêtre matinale prédéterminée par la patiente; en mode TCB l'instrument possède également une fenêtre d'acceptation des températures: 35,0°C—37,5°C.

Pour mesurer la TCB l'appareil est relié à une sonde NTC, l'extrémité de cellei-ci étant placée sous la langue de la patiente; le mode d'utilisation est le suivant:

(i) Le début du premier cycle est indiqué à l'appareil en pressant le code AAA (mode TCB) à l'aide des boutons pressoirs. Cette opération provoque un reset général et indique à l'appareil que ce jour est le jour no. 1 du cycle; cette opération, faite le premier jour au réveil, fixe l'heure "idéale" pour la mesure journalière; à partir de ce pointeur, l'instrument fixe lui-même une fenêtre journalière de 4 heures (2 heures de part et d'autre du pointeur) pour l'acceptation d'une température. Dès le jour no. 2 et jusqu'à la fin du cycle, un signal sonore, placé exactement au temps du pointeur, indiquera à la patiente qu'il faut procéder à une mesure.

(ii) La sonde NTC est placée dans la bouche, sous la langue. Dès que la température de la sonde dépasse un seuil fixé, l'instrument se réveille automatiquement et se met à mesurer la TCB d'une manière similaire à un thermomètre à mercure (thermomètre à maximum). Lorsque la température est acceptée par l'appareil, un signal sonore avertit la patiente que la mesure est terminée; l'appareil se ''réendort'' automatiquement.

(iii) Les températures des 3 premiers jours du cycle sont stockées, mais ne sont pas prises en considération pour l'analyse de la courbe. Le calculateur accepte ensuite encore 5 températures journalières (jours no. 4-5-6-7-8) avant de commencer l'analyse de la courbe en utilisant l'algorithme no. 1. Une fois la température du jour no. 8 stockée, l'instrument calcule:

(1) $M_F$ = moyenne arithmétique des jours 4 et 5
(2) $M_L$ = moyenne arithmétique des jours 6—7 et 8
(3) $D = M_F - M_L$

Si $D > 0,3$, alors le premier des 3 jours ayant constitué la moyenne $M_L$ (ici le jour no. 6) est supposé être le jour d'ovulation (0).

Si, au contraire, $D < 0,3$ alors le calculateur attend la prochaine température et re-calcule D entre la moyenne $M_L$ des 3 derniers jours du cycle entrés (maintenant 7-8-9) et celle $M_F$ de tous les jours précédents (maintenant 4-5-6) et ainsi de suite jusqu'à ce que D soit supérieur à 0,3°C.

(iv) Lorsque D est $> 0,3$, l'antépénultième jour du cycle est supposé être le jour d'ovulation (jour 0). Une fois le jour 0 trouvé, l'algorithme no. 2 est utilisé pour les jours suivants: la moyenne de la phase folliculaire (phase postmenstruelle) supposée $M_F$ ne varie plus; par contre la moyenne de la phase lutéale (phase prémenstruelle) supposée $M_L$ est dès lors calculée en utilisant tous les jours du cycle à partir du jour 0 et varie donc avec chaque température nouvellement introduite. Le jour 0 est confirmé jour d'ovulation si $D = M_L - M_F$ resté supérieur ou égal à 0,2°C pendant au moins 3 jours (c'est-à-dire jusqu'au jour 0+6). Au cas où D redevient inférieur à 0,2°C avant le jour 0+6, l'algorithme no. 1 est alors à nouveau utilisé et la recherche d'un nouveau jour d'ovulation recommence.

(v) Si un jour est sauté ou si la température d'un jour est supérieure à 37,5°C (fièvre), la température de ce jour est remplacée par la moyenne du jour qui le précède et celle du jour qui le suit immédiatement.

(vi) En utilisant les algorithmes no. 1 et no. 2, il est parfaitement possible d'arriver à la fin d'un cycle sans avoir observé ni fixé de jour d'ovulation. Dans une telle situation, et à la fin du cycle uniquement, l'appareil utilise un algorithme no. 3 qui, ayant à disposition toutes les températures du cycle, procède à la recherche d'un cycle bi ou monophasique à l'aide du schéma mathématique suivant:

(1) Le cycle est arbitrairement divisé en 4 parties égales I, II, III et IV et la température moyenne de chacune de ces 4 parties est calculée $M_I$, $M_{II}$, $M_{III}$ et $M_{IV}$. Ces 4 moyennes sont ensuite comparées à la moyenne générale du cycle M. Il existe dès lors 16 possibilités de combinaisons mathématiques représentées par le tableau suivant:

| $M_I$ | $M_{II}$ | $M_{III}$ | $M_{IV}$ | no. | commentaires |
|-------|----------|-----------|----------|-----|--------------|
| + | + | + | + | 1 | impossible |
| + | + | + | − | 2 | irrégulier ou monophasique |
| + | + | − | + | 3 | biphasique à confirmer ($M_{IV}$) |
| + | + | − | − | 4 | irrégulier/ monophasique |
| + | − | + | − | 5 | biphasique à confirmer ($M_{III}$) |
| + | − | − | + | 6 | biphasique à confirmer ($M_{IV}$) |
| + | − | − | − | 7 | irrégulier/ monophasique |
| + | − | + | + | 8 | biphasique |
| − | + | + | + | 9 | biphasique |
| − | − | + | + | 10 | biphasique |
| − | + | + | − | 11 | biphasique |
| − | − | + | − | 12 | biphasique à confirmer ($M_{III}$) |
| − | + | − | + | 13 | biphasique à confirmer ($M_{IV}$) |
| − | − | − | + | 14 | biphasique |
| − | + | − | − | 15 | irrégulier/ monophasique |
| − | − | − | − | 16 | impossible |

+=$M_{I, II, III}$ ou $_{IV}$ supérieur à M
−=$M_{I, II, III}$ ou $_{IV}$ inférieur à M.

Les situations extrèmes (1 et 16) sont impossibles. Les situations 2-4-7-15 sont fort probablement caractéristiques de courbes irrégulières (monophasiques). Les situations 3-5-6-12-13 sont peut-être biphasiques mais nécessitent un algorithme de confirmation. Enfin, les situations 8-9-10-11-14 sont fort probablement biphasiques.

(2) Dans les cas où nous sommes confrontés à une courbe irrégulière ou monophasiques (2-4-7-15), ceci est mentionné sur le display à l'attention du médecin gynécologue; on peut ensuite recommencer un nouveau cycle sans pour autant perdre les informations concernant le cycle précédent.

(3) Dans les cas où nous sommes confrontés à une courbe biphasique (8-9-10-11-14), un algorithme no. 4 est alors utilisé pour la recherche du jour d'ovulation: en évoluant le long du cycle, on recherche la première température journalière qui est au-dessus de la moyenne générale M du cycle: jour $P_1$. Puis, à partir du jour $P_1$, on recherche quelle est la prochaine température qui est au-dessous de M: jour $P_2$. Si $D_1=P_2-P_1$ est plus petit que 5 jours, alors $P_1$ n'est pas considéré comme le jour d'ovulation 0 et le processus de recherche recommence. Au contraire, si $D=P_2-P_1$ est supérieur à 5 jours ou si $P_2$ coincide avec l'antépénultième, l'avant-dernier ou le dernier jour de cycle, alors $P_1$ est considéré comme le jour d'ovulation.

(4) Dans le cas où nous sommes confrontés à une courbe biphasique à confirmer (3-5-6-12-13), l'algorithme no. 4 est aussi utilisé. Lorsque le jour d'ovulation 0 est trouvé, un algorithme no. 5 de confirmation contrôle si le jour d'ovulation trouvé se situe bien dans un quadrant est $M_{III}$ ou $M_{IV}$. Si les deux conditions ne sont pas satisfaites, le jour 0 est rejeté et l'inscription "courbe irrégulière" est affichée.

(vii) Dans le cas où le calculateur a observé un jour 0 lors de l'introduction des résultats, il stocke cette information en mémoire. A la fin du cycle, il recherche à nouveau le jour d'ovulation en utilisant les algorithmes no. 3 et 4 (décrit sous (vi)).

Si ce jour 0 correspond au jour d'ovulation (+ ou − 2 jours) trouvé à l'aide des algorithmes no. 1 et 2, alors le jour 0 est confirmé définitivement. Dans le cas contraire, on utilise à nouveau l'algorithme de confirmation no. 5 pour accepter ou rejeter le jour d'ovulation 0 (cf. point (4) paragraphe (vi)).

(viii) En cas d'ovulation confirmé définitivement, on peut afficher sur le display: la longueur du cycle, le jour 0 et les longueurs de phases folliculaires et lutéales. Toutes ces valeurs sont stockées en mémoire à des fins de prévision pour les cycles futurs.

(ix) En présence d'une courbe irrégulière ou monophasique, le gynécologue a la possibilité de tenter de réinitier un cycle biphasique en appliquant un traitement hormonal à sa patiente.

Etant donné qu'un traitement hormonal suit en général un schéma classique, le calculateur indiquera à la patiente quand celle-ci doit subir son traitement hormonal et quand elle doit se rendre chez le médecin pour un contrôle physique ou un prélèvement sanguin.

L'appareil peut aussi indiquer au couple désirant avoir une conception quelle est la période de fertilité maximale. Cette prévision utilise les informations stockées au cours de cycles précédents.

L'instrument possède encore une sortie série normalisée qui permet, soit de transférer les températures du cycle sur un ordinateur plus puissant et donc de réaliser une analyse plus complète de la courbe, soit d'imprimer les températures sur une imprimante de poche et d'y imprimer sommairement la courbe mensuelle.

Le calculateur peut être utilisé comme une montre et un calendrier digital, et comme un thermomètre médical. On peut accéder à tout moment à ces informations en utilisant des fonctions spéciales qui n'altèrent pas le fonctionnement de l'instrument en tant que calculateur électronique utilisé pour le traitement de l'infertilité féminine. Il découle de ce qui est décrit ci-dessus que l'appareil peut aussi être utilisé dans le domaine du planning familial.

La calculatrice électronique développée spécialement pour le contrôle et le traitement de l'infertilité est réalisée à partir d'un microordinateur intégré sur une seule pastille de silicium. L'appareil (voir fig. 1), montrant un exemple d'éxécution de l'instrument selon l'invention, est composé des éléments principaux suivants:

Mesure

La sonde de mesure 11 est une résistance à coéfficient de température négatif (CTN). Elle est encapsulée dans un boîtier approprié à la prise de température buccale. La sonde fait partie d'un pont de mesure 12 de Wheatstone. La différence de potentiel 14 créé par un déséquilibre du point est convertie en un nombre binaire par un convertisseur analogique-numérique 15 double rampe. Celui-ci intègre, pendant une durée constante, la tension mesurée en diagonale du pont 14 et ensuite décharge sont intégrateur avec uné tension proportionnelle 13 à la tension d'alimentation du pont. Il en résulte que le nombre binaire 18, résultant de la conversion, ne dépend que du rapport des résistances du point. D'autre part, les résistances du point sont choisies de telle façon que seul un étalonnage du point minimum de la plage de mesure soit nécessaire. Cet étalonnage est ici réalisé digitalement à l'aide d'une entrée binaire 16, le point ne comportant aucune résistance variable.

Spécifications:

— plage de mesure 35°C à 40°C
— résolution+0,05°C
— précision absolue+0,1°C.

Commande

Un clavier numérique composé de douze touches permet d'introduire des données et de mettre l'appareil dans les différents modes de fonctionnement. L'introduction de données ou de commandes à partir du clavier est réalisée par une séquence de pressions sur les touches. Chaque commande est représentée par un code qui est visualisé sur l'affichage.

Affichage

L'appareil est muni d'un affichage alphanumérique, composé d'éléments LCD 31, connecté au microordinateur par l'intermédiaire d'un contrôleur d'affichage 32 réalisant le démultiplexage. Il est capable d'afficher aussi clairement que possible:

— les entrées ou pressions sur le clavier
— la température mesurée
— les données d'une horloge
— les résultats des analyses et les contraintes du traitement
— le contenu de la mémoire.

Horloge

L'appareil est muni d'un circuit d'horloge 41 autonome capable à partir d'un quartz 43, de fournir en tout temps au processeur, respectivement à l'utilisateur, la date complète et l'heure. Dans le fonctionnement normal de l'appareil, l'horloge est surtout consultée pour dater les prises de température journalières qui ne sont en outre admises que durant une limite de quatre heures. D'autre part, l'horloge permet de signaler à la patiente:

— l'heure de la prise de température journalière par une sonnerie sur le vibreur piezoélectrique

— les actions journalières à entreprendre dans le cas d'un traitement.

Une pile 42 garantit un fonctionnement sans interruption de l'horloge.

## Micro-ordinateur

L'appareil comprend un micro-ordinateur constitué des élements suivants:

— unité centrale 51 assurant la gestion du système
— mémoire morte 52 qui contient l'ensemble du programme gérant le fonctionnement de l'appareil et de l'ensemble du programme de traitement
— mémoire vive 53: ce sont des mémoires à semiconducteurs CMOS dont la tension d'alimentation est gardée en permanence par les batteries du système d'alimentation. Cette mémoire permet l'enregistrement des contraintes du traitement de l'infertilité et l'ensemble des résultats intermédiaires résultants des analyses précédentes,
— unités de communication: circuiterie permettant de connecter l'ensemble des éléments périphériques au micro-ordinateur 54.

## Sortie série

Une circuiterie permet à l'utilisateur de sortir l'ensemble des données contenues dans les mémoires d'une part vers d'autres calculateurs par une sortie série normalisée 61 et d'autre part 62 vers une mini-imprimante thermique de façon à obtenir un document sur les résultats de l'analyse.

## Vibreur piézo-électrique

L'appareil comprend un vibreur piézo-électrique contrôlé par le micro-ordinateur permettant de signaler de façon acoustique les événements importants dans le fonctionnement de l'appareil:

— heure de prise de température
— fin de la prise de température
— événement important pour le traitement.

## Alimentation

L'alimentation de l'appareil est assurée par un circuit redresseur filtré 86 connecté sur le réseau d'alimentation. Pour les pannes de réseau, les périodes de déconnection accidentelle ou pour le déplacement de l'appariel, les fonctions principales (mémoire vive et horloge) sont maintenues sous tension à l'aide de batteries 81 qui sont automatiquement maintenues chargées par la connection sur le réseau. Si l'alimentation réseau manque, seul un certain nombre de circuits importants sont maintenus sous tension, les autres sont déconnectés au moyen de déclencheur 85.

## Revendications

1. Instrument électronique pour le contrôle et le traitement de l'infertilité féminine comprenant une sonde thermistor (11) pour la mesure de la température, un circuit d'entrée, reliant la sonde (11) à un microprocesseur (5), comportant un convertisseur analogique-numérique (15) permettant de transformer la variation de résistance du thermistor en bits, un circuit (41, 42, 43) d'horloge permettant de n'accepter qu'une température par jour dite température corporelle basale (TCB), un dispositif (7) acoustique signalant le début et la fin de la prise de température, le microprocesseur étant agencé pour faire une analyse continue et globale de la courbe mensuelle des températures corporelles basales, et un display (31), instrument électronique étant caractérisé en ce que le microprocesseur comporte une mémoire RAM pour le stockage non volatile de 100 températures corporelles basales, en ce que le dispositif acoustique est un vibreur piezo-électrique, en ce que le display est un display LCD commandé par l'extérieur et en ce que ledit instrument électronique comporte des moyens discriminateurs pour remplacer une température corporelle basale manquante correspondant à un jour sauté ou une température corporelle basale trop élevée correspondant à un jour de fièvre par la moyenne des températures corporelles basales des 2 jours entourant le jour sauté ou le jour de fièvre et des moyens comparateurs permettant de comparer, dès le 8ème jour du cycle, deux moyennes de température: une première fois la moyenne $M_F$ de la phase folliculaire supposée, dans les jours 4 et 5 du cycle, et la moyenne $M_L$ de la phase lutéale supposée, dans les jours 6, 7 et 8 du cycle, les 3 premiers jours du cycle n'étant pas pris en considération, ces moyens comparateurs étant tels que ce mécanisme de comparaison se poursuive d'autres fois à chaque prise de température jusqu'à ce que $D=M_L-M_F$ dépasse un niveau prédéterminé, $M_L$ correspondant à la moyenne des trois dernières températures corporelles basales et $M_F$ à la moyenne des autres températures à partir du quatrième jour, après quoi on considère d'être en présence de la seconde phase du cycle, l'antipénultième jour du cycle étant alors considéré comme $ETO_s$, d'est-à-dire l'"Estimated Time of Ovulation".

2. Instrument selon la revendication 1, comportant, en outre, des moyens permettant, à la fin du cycle, de classifier la courbe mensuelle dans l'une des 3 catégories suivantes:

(i) courbe mensuelle monophasique ou irrégulières des températures corporelles basales,
(ii) courbe mensuelle supposée biphasique
(iii) courbe mensuelle biphasique certaine.

3. Instrument selon la revendication 2, comportant, en outre, des moyens pour classifier les courbes supposées biphasiques en "courbes monophasiques" ou en "courbes biphasiques".

4. Instrument selon la revendication 2, comportant, en outre, des moyens permettant de calculer l'ETO$_f$, c'est-à-dire l'"'Estimated Time of Ovulation—final" dans le cas d'une courbe classée "biphasique".

5. Instrument selon la revendication 4, comportant, en outre, des moyens pour comparer l'ETO$_s$ et l'ETO$_f$ afin d'accepter ou de rejeter l'ETO, c'est-à-dire l'"'Estimated Time of Ovulation".

6. Instrument selon la revendication 1, comportant, en outre, des moyens d'afficher sur le display LCD (31) le jour de l'ETO, la longueur du cycle, ainsi que les longueurs des phases post- et prémenstruelles, et des moyens permettant de mémoriser ces valeurs.

7. Instrument selon la revendication 1, comportant, en outre, des moyens pour faire défiler sur le display LCD (31) chacune des 100 températures journalières mémorisées, accompagnées des pointeurs de début du cycle, c'est-à-dire 1er jour des règles, des pointeurs de jour sauté et des pointeurs de jour de fièvre.

8. Instrument selon la revendication 1, comportant, en outre, des sorties (61) normalisées permettant le transfert automatique des températures journalières mémorisées dans un ordinateur plus puissant du microprocesseur à des fins d'analyse détaillée, le transfert et l'impression automatique des températures journalières mémorisées sur une imprimante (62), le transfer et l'expression graphique de la courbe mensuelle sur l'imprimante (62), l'impression du code de la patiente, du jour de l'ETO, de la longueur du cycle et des phases post- et prémenstruelles sur l'imprimante et le transfert et l'impression de planning journalier personalisé pour la thérapie hormonale.

9. Instrument selon la revendication 1, comprenant 12 boutons pressoirs permettant, grâce à un nombre de code défini, d'accéder

avec le code AAA à la fonction TCB, c'est-à-dire mesure de la température corporelle basale,

avec le code BBB à la fonction T, c'est-à-dire traitement,

avec le code CCC à la fonction thermomètre, c'est-à-dire simple mesure de la température,

avec le code DDD à la fonction horloge, c'est-à-dire affichage des heures, minutes et secondes et

avec le code EEE à la fonction calendrier, c'est-à-dire affichage des années, mois et jours.

10. Instrument selon la revendication 1, comprenant, en outre, des moyens permettant à l'instrument de fonctionner comme un thermomètre à maximum.

11. Instrument selon la revendication 1, le display LCD (31) indiquant, en plus, à l'utilisatrice le numéro du jour du cycle et sa température associée.

12. Instrument selon la revendication 1, dans lequel le circuit d'horloge permet de n'accepter cette température corporelle basale qu'à l'intérieur d'un intervalle de temps fixé, dit fenêtre matinale, et de n'accepter cette température corporelle basale que si elle est située entre 35,0 et 37,5°C.

**Patentansprüche**

1. Elektronisches Instrument für die Kontrolle und die Behandlung der weiblichen Infertilität, das Instrument aufweisend eine Thermistorsonde (11) für die Temperaturmessung, eine Daten-Eintrittsschnittstelle zur Daten-Uebertragung von der Sonde zum Mikroprozessor (5) mit einem Analog/Digital-Umformer (15) zur Umwandlung der Widerstandsänderung des Thermistors in Bits, einen Kreislauf (41, 42, 43) mit Uhr zur Sicherstellung, dass pro Tag nur eine Eingabe der Basal-Körpertemperatur (TCB) erfolgt, eine Akustikvorrichtung (7), welche Beginn und Ende der Zeitperiode, in der die Temperaturmessung zu erfolgen hat, anzeigt, wobei der Mikroprozessor zwecks kontinuierlicher und umfassender Analyse der Perioden-TCB-Kurve programmiert ist, und eine LCD-Anzeige (31),

das genannte elektronische Instrument dadurch gekennzeichnet,

dass der Mikroprozessor einen RAM-Speicher zur dauernden Speicherung von 100 TCB-Werten aufweist (constant memory),

dass die Akustikvorrichtung ein piezoelektrischer Vibrator ist,

dass die Anzeige ein LCD-Display ist mit Zugriff und Eingabemöglichkeit von aussen und

dass es Mittel zur Ersetzung einer nicht erfolgten oder zu hohen TCB-Eingabe durch den Mittelwert der TCB-Messungen des Vor- und Nachtages sowie Mittel zum Vergleich, ab dem achten Tag der Periode, des Mittelwertes $M_F$. d.h. des Mittels der TCB-Werte der Tage 4 und 5 in der angenommenen Follikularphase mit dem Mittelwert $M_L$, d.h. des Mittels der TCB-Wert der Tage 6, 7 und 8 in der angenommenen Lutealphase, wobei die Messungen der ersten drei Tage der Periode unberücksichtigt bleiben und wobei der genannte Vergleich weiter erfolgt, bis $D = M_L - M_F$ einen vorgegebenen Wert übersteigt, wobei dann $M_L$ dem Mittelwert der drei letzten TCB-Eingaben und $M_F$ den vorherigen TCB-Eingaben vom 4. Tag der Perioden entsprechen, worauf angenommen wird, dass die zweite Phase der Periode eingesetzt hat und der vor-vorletzte Tag des Messzyklus der Tag der ETO$_S$ (Estimated Time of Ovulation supposé) ist, aufweist.

2. Elektronisches Instrument gemäss Patentanspruch 1, weiter umfassend Mittel zur Klassifizierung der Perioden-Temperaturkurve—am Schluss des Zyklus—in eine der folgenden Kategorien:

(i) Monophasische oder unregelmässige TCB-Kurve,

(ii) als biphasisch angenommene TCB-Kurve oder

(iii) bestimmt biphasische TCB-Kurve.

3. Elektronisches Instrument gemäss Patentanspruch 2, weiter umfassend Mittel zur Klassifizierung der anscheinend biphasischen Kurven in monophasische oder in bestimmt biphasische.

4. Elektronisches Instrument gemäss Patentanspruch 2, weiter umfassend Mittel zur Berechnung von $ETO_f$, d.h. "Estimated Time of Ovulation—final", im Falle einer bestimmt biphasischen TCB-Kurve.

5. Elektronisches Instrument gemäss Patentanspruch 4, weiter umfassend Mittel zum Vergliech von $ETO_s$ und $ETO_f$ zwecks Annahme oder Verwerfung von ETO, d.h. von "Estimated Time of Ovulation".

6. Elektronisches Instrument gemäss Patentanspruch 1, weiter umfassend Mittel zur Anzeige auf dem LCD-Display (31) des ETO-Tages der Dauer der Periode und der Längen der prä- bzw. postmenstruellen Phasen, sowie Mittel, um diese Werte zu speichern.

7. Elektronisches Instrument gemäss Patentanspruch 1, weiter umfassend Mittel zur gelisteten Anzeige auf dem Display (31) der 100 gespeicherten Tages-Temperaturen zusammen mit den Zeigern betreffend Periodenbeginn f, d.h. erster Tag der Menstruation, den Zeigern betreffend Tage ohne Messung und den Zeigern betreffend Tage mit zu hohen Temperaturen, praktisch Fiebertagen.

8. Elektronisches Instrument gemäss Patentanspruch 1, weiter umfassend Standard-Schnittstellen (61), welche die Uebertragung

von Daten vom Mikroprozessor auf ein leistungsfähiges EDV-System zur detaillierteren Analyse derselben,

von Daten auf Druckersysteme zur Darstellung von Listen und Kurven,

vom Patientencode mit ETO-Tag, Dauer der Periode und der prä- und postmenstruellen Phasen auf Druckersysteme und

von Daten für die Hormonbehandlung betreffend Patienten ermöglichen.

9. Elektronisches Instrument gemäss Patentanspruch 1, weiter umfassend 12 Tasten, um gemäss bestimmten Codes die folgenden Funktionen zu aktivieren:

AAA: Funktion TCB, d.h. tägliche Messung der Basal-Körpertemperatur,

BBB: Funktion B, d.h. Behandlung,

CCC: Funktion T, d.h. Thermometer,

DDD: Funktion H, d.h. Uhr und

EEE: Funktion K, d.h. Kalender.

10. Elektronisches Instrument gemäss Patentanspruch 1, weiter umfassend Mittel, die die Verwendung des Instrumentes als Maximum-Thermometer gestatten.

11. Elektronisches Instrument gemäss Patentanspruch 1, bei dem der LCD-Display (31) zusätzlich den Tag der Periode und die dazugehörige Körpertemperatur anzeigt.

12. Elektronisches Instrument gemäss Patentanspruch 1, bei dem der Kreislauf mit Uhr die Eingabe der Temperaturmessung nur innerhalb eines bestimmten Zeitfensters und eines Temperaturbereiches zwischen 35,0 und 37,5°C erlaubt.

**Claims**

1. An electronic instrument for the control and treatment of infertility in women, comprising a thermistor probe (11) for measuring temperature, an input circuit connecting the probe (11) to a microprocessor (5) which comprises an analogue-to-digital converter (15) for transforming the variation in resistance of the thermistor into bits, a timing circuit (41, 42, 43) which will accept only one temperature per day, known as the Basal Body Temperature (BBT), an acoustic device (7) which signals the start and finish of the temperature taking; the microprocessor being designed to effect continuous total analysis of the monthly curve of basal body temperatures, and a display (31), this electronic instrument being characterised in that the microprocessor comprises a RAM memory for non-volatile storage of 100 basal body temperatures, the acoustic device is a piezoelectric vibrator, the display is an externally controlled LCD display and the electronic instrument comprises discriminating means for replacing a missing basal body temperature, corresponding to a day omitted, or too high a basal body temperature, corresponding to a day of fever, by the mean of the basal body temperatures of the 2 days either side of the day omitted or the day of fever; and comparing means for comparing, from the 8th day of the cycle, two average temperatures $M_F$ and $M_L$ wherein $M_F$, on the first occasion, is the mean of the supposed follicular phase, on the 4th and 5th days of the cycle and ML, on the first occasion, is the mean of the supposed luteal phase, on days 6, 7 and 8 of the cycle, the first 3 days of the cycle being disregarded, the comparing means being such that the comparison mechanism is repeated on subsequent occasions each time the temperature is taken until the value of $M_L-M_F$, D, exceeds a predetermined level, $M_L$ corresponding to the mean of the last three basal body temperatures and $M_F$ corresponding to the mean of the other temperatures starting from the 4th day, after which the second phase of the cycle is considered to be under way, the ante-penultimate day of the cycle then being considered as the Estimated Time of Ovulation—Suppose $ETO_s$.

2. An instrument as claimed in claim 1, further comprising means for classifying the monthly curve at the end of the cycle into one of the following 3 categories:

(i) a monophasic or irregular monthly curve of basal body temperatures,

(ii) a supposedly biphasic monthly curve

(iii) a definitely biphasic monthly curve.

3. An instrument as claimed in claim 2, further comprising means for classifying supposedly biphasic curves as "monophasic curves" or "biphasic".

4. An instrument as claimed in claim 2, further comprising means for calculating the Estimated Time of Ovulation—final, $ETO_f$, in the case of a curve classed as "biphasic".

5. An instrument as claimed in claim 4, further comprising means for comparing the ETO$_s$ and the ETO$_f$ in order to accept or reject the Estimated Time of Ovulation, ETO.

6. An instrument as claimed in claim 1, further comprising means for displaying, on the LCD display (31), the day of the ETO, the length of the cycle and the length of the post- and pre-menstrual phases, and means for memorising these values.

7. An instrument as claimed in claim 1, further comprising means for indicating on the LCD display (31) each of the 100 memorised daily temperatures accompanied by pointers for the start of the cycle, i.e. the first day of the period, pointers for missed days and pointers for days of fever.

8. An instrument as claimed in claim 1, further comprising normalised output data (61) permitting automatic transfer of the daily temperatures memorised to a more powerful computer than the microprocessor for the purposes of detailed analysis, transfer and automatic printing of the memorised daily temperatures on a printer (62), transfer and graphic expression of the monthly curve on the printer (62), printing of the patient's code, the day of the ETO, the length of the cycle and the post- and premenstrual phases on the printer and the transfer and printing of personalised daily planning for hormone therapy.

9. An instrument as claimed in claim 1, comprising 12 push-buttons in a housing by means of which, through the use of a specified code number, it is possible to access, with the code AAA the function BBT, i.e. a measurement of the basal body temperature, with the code BBB, the function T, i.e. treatment, with the code CCC, the thermometer function, i.e. a simple measurement of temperature, with the code DDD, the clock function, i.e. a display of hours, minutes and seconds and with the code EEE, the calender function, i.e. a display of years, months and days.

10. An instrument as claimed in claim 1, further comprising means enabling the instrument to operate as a maximum thermometer.

11. An instrument as claimed in claim 1 wherein the LCD display (31) further indicates to the user the number of the day of the cycle and the associated temperature.

12. An instrument as claimed in claim 1 wherein the clock circuit ensures that the basal body temperature will only be accepted within a fixed time interval, the timing window, and will accept this basal body temperature only if it is within the range of from 35.0 to 37.5°C.

Clavier de commande 12 touches

2

31 Affichage LCD

32 Contrôleur d'affichage

3

Etalonnage 16

12 Pont de mesure

13 référence

U(T) 14

11 Sonde

A 15 N

18

1

54

Entrées-sorties du micro-ordinateur

Bus d'adresse 5

CPU 51

52 PROM ou ROM mémoire morte

53 RAM mémoire vive

43

41 Horloge

42

4

Vibreur piezo alarme 7

Bus de données

Bus de contrôle

Sortie série RS232 61

Sortie pour imprimante

62

6

Alimentation permanente

Alimentation déclenchable

Alimentation 85

Accumulateurs 81 4×1,2 V

86 220V 50 Hz ~

8

**Fig. 1**

Schema de l'appareil